(19) **Europäisches Patentamt**

**European Patent Office**

**Office européen des brevets**

(11) **EP 1 171 841 B1**

(12) **EUROPEAN PATENT SPECIFICATION**

(45) Date of publication and mention
of the grant of the patent:
**16.04.2003 Bulletin 2003/16**

(51) Int Cl.$^7$: **G06F 19/00**

(21) Application number: **00910117.1**

(86) International application number:
**PCT/US00/03318**

(22) Date of filing: **08.02.2000**

(87) International publication number:
**WO 00/065523 (02.11.2000 Gazette 2000/44)**

(54) **SYSTEM AND METHOD FOR MODELLING GENETIC, BIOCHEMICAL, BIOPHYSICAL AND ANATOMICAL INFORMATION**

SYSTEM UND VERFAHREN ZUR MODELLIERUNG VON GENETISCHE, BIOCHEMISCHE, BIOPHYSISCHE UND ANATOMISCHE INFORMATION

SYSTEME ET PROCEDE DE MODELISATION D'INFORMATIONS GENETIQUES, BIOCHIMIQUES, BIOPHYSIQUES ET ANATOMIQUES

(84) Designated Contracting States:
**AT BE CH CY DE DK ES FI FR GB GR IE IT LI LU MC NL PT SE**

(30) Priority: **21.04.1999 US 295503**

(43) Date of publication of application:
**16.01.2002 Bulletin 2002/03**

(73) Proprietor: **Physiome Sciences, Inc.**
**Princeton, NJ 08540 (US)**

(72) Inventors:
• **WINSLOW, Raimond, Lester**
**Timonium, MD 21093 (US)**
• **JAFRI, Moshin, Saleet**
**Richardson, TX 75081 (US)**
• **GREHLINGHER, Peter, Mark**
**Allentown, NJ 08501 (US)**
• **LI, Jian**
**Plainsboro, NJ 08536 (US)**

(74) Representative: **Haley, Stephen**
**Gill Jennings & Every,**
**Broadgate House,**
**7 Eldon Street**
**London EC2M 7LH (GB)**

(56) References cited:
**US-A- 5 657 255**

• **DAFOE ET AL: "IN SILICO KNOWLEDGE DISCOVERY IN BIOMEDICAL DATABASES" PROCEEDINGS OF THE FIFTH WORKSHOP ON NEURAL NETWORKS: WNN93/FNN93, 7 November 1993 (1993-11-07), pages 239-243, XP000197711 San Diego, CA, US**
• **FINK ET AL: "Modeling disease processes for drug development: bridging the gap between quantitative and heuristic models" PROCEEDINGS OF THE 1996 WINTER SIMULATION CONFERENCE, 8 - 11 December 1996, pages 1183-1190, XP002076640 Coronado, CA, US ISBN: 0-7803-3383-7**
• **SANDBLAD ET AL: "Modelling and simulation of complex control structures in cell biology" METHODS OF INFORMATION IN MEDICINE, vol. 31, no. 1, February 1992 (1992-02), pages 36-43, XP002109983 DE ISSN: 0026-1270**

Description

## BACKGROUND OF THE INVENTION

### 1. Field of the Invention:

The present invention relates to a computer-implemented system of constructing databases and modeling biological processes; and more particularly to mathematical, informational, and computational processes and procedures for automatically generating computer-based models that integrate biological information from the subcellular to the cellular level.

### 2. Description of the Prior Art:

The amount of biological information continues to amass exponentially. At present, hundreds of biological databases are listed in DBCAT, the INFOBIOGEN biological database catalog accessible from the World Wide Web (http://www.infobiogen.fr/services/dbcat/) and available publicly through the National Center for BioTechnology Information (http://www.ncbi nlm.nih.gov) . This information explosion has been driven by the continuous development of information technology such as the Internet as well as the development of powerful new technologies for automatically collecting and storing data such as in gene sequencing and gene expression profiling. These databases contain genomic, biochemical, chemical and molecular biology data as well as structural databases that contain geometric and anatomical information from the subcellular to the whole organism level. Some of these data are organized by data type including, for example, the International Nucleic Acid Sequence Data Library (a.k.a. GenBank) and NAD for nucleic acid sequences; SWISS-PROT for protein sequences; PDB for protein structures and the like. Other databases are organism specific and include GDB and OMIM for human; MGD for mouse, PigBASE for pig; AtDB for Arabidopsis; ECDC for E. Coli, and many others. Other databases contain information on particular areas of interest, such as specific databases for individual genes, databases about specific protein families, databases of transcription factors and the like. Biochemical databases contain information regarding coupled biochemical reactions and feedback signals which take place within the cell. Additionally, proprietary databases such as those available from the large data production houses have been created and are expanding with technology; such as the availability of entire genomic sequences due to improved high throughput gene sequencing. Substantial work is underway to integrate data from these diverse databases. *See e.g..* Macauley, et al., A Model System for Studying the Integration of Molecular Biology Databases, 14 *Bioinformatics* 575-582 (1998).

Efforts to organize and analyze the vast amount of genomic data has stimulated the development of a new field of computational science known as bioinformatics; the science of using computers and software to store, extract, organize, analyze, interpret and utilize gene sequence data to identify new genes and gene function in order to understand the genetic basis of disease and to further gene-based drug discovery and development. This approach typically uses a one-dimensional computational analysis to study explicit information about the genome such as percentage of gene sequence similarity across species, homology of sequence motifs across species, expression levels in various tissue types, secondary structure correlations, etc. Although the acquisition of genomic information is clearly essential, there is growing recognition that the current methods are insufficient for correlating that information with the functional role of genes and gene products. Rather, in all cells, genetic expression produces self-organizing networks controlling cell functions, including developmental pathways, progression through cell cycle, metabolism, intracellular signaling, cell excitability and motility, and feedback loops regulating gene expression. At present, bioinformatics is unable to simulate these complex, highly nonlinear dynamic interactions that occur between each gene or gene product, and other components of the network they are a part of. Thus, bioinformatics researchers do not, at present, have the necessary tools to obtain a complete representation of cellular and subcellular processes.

One approach to dealing with these complex, highly nonlinear interactions has focused on computational modeling. There is an extensive 40 year history of such modeling that includes simple models with a few state equations that describe processes within cells to highly complex models of organ systems that must be implemented on high performance multiprocessor computers (Rail W, Burke RE, Holmes WR, Jack JJ, Redman SJ, Segev I (1992). Physiol. Rev. 72(4 Suppl) 5159-86; Rall W (1967) J Neurophysiol 30(5): 1169-93; Segev I and Rall W (1998) Trends Neurosci 21(11): 453-60; Koch C, Poggio T, and Torre V (1982) Philos Trans Roy Soc Lond B 298(1090):227-63; Chay TR and Rinzel J (1985) Biophys J 47(3): 357-66; Smolen P, Rinzel J, Sherman A (1993) Biophys J 64(6): 1668-80; Shepherd GM et al (1998) Trends Neurosci 21(11): 460-8). This approach provides a means to link experimental data regarding specific biological processes to cell function. The culmination of this 40 year history can be seen in several efforts such as the nationally funded efforts, The Human Brain Project and the Virtual Cell Project. The Human Brain Project is a multi-agency funded multi-site effort to organize and utilize diverse data about the brain and behavior. The Virtual Cell project has developed a framework for organizing, modeling, simulating, and visualizing cell structure and physiology.

However, these projects lack an overall ability to link to existing genetic, protein and structural data bases. In addition, these projects have not defined procedures for modeling biological systems using information stored in local or distributed databases. As such, detailed and accurate representations of the many different simultaneous subcellular and cellular processes which occur at any given time are not presently possible.

What is needed therefore are new computer based tools for the database storage of information needed to formulate computational models of subcellular and cellular processes, and for coupling this database information to tools for formulating, simulating and analyzing such models. Such tools will provide a means for linking information at the level of the gene to functional properties of cells in health and disease, will further the understanding of disease processes. and aid in drug target identification and screening.

## SUMMARY OF THE INVENTION

In accordance with the present invention, there is provided a system and method for constructing computer-executable models of the dynamic behaviour of biological processes, thereby, integrating genetic, biochemical, biophysical and anatomical information at the subcellular and cellular level.

Advantageously, the system of the present invention can access and tabulate genetic information contained within proprietary and nonproprietary databases, combine this with functional information on the biochemical and biophysical role of gene products and based on this information formulate, solve and analyze computational models of genetic, biochemical and biophysical processes within cells. The system of the invention therefore provides a dynamic tool for quantitative understanding of biological processes, identifying new drug targets for therapeutic intervention and predicting the outcome of drug screening. This is accomplished by the accurate modeling and simulation of highly complex nonlinear dynamic interactions that occur between each gene or gene product.

In another aspect of the invention there is provided a computer-executable model as constructed with the inventive system. The models created by the system integrate biological knowledge across all levels of analysis ranging from that of the gene to that of the cell to provide a detailed and accurate representation of the system being studied. This integration provides a multi-dimensional analysis which simply was not possible with the one-dimensional genomic computational analysis tools of the prior art.

In yet another aspect of the present invention there is provided a method of storing and searching biological information in the inventive system.

## BRIEF DESCRIPTION OF THE DRAWINGS

The invention will be more fully understood and further advantages will become apparent when reference is made to the following detailed description and the accompanying drawings in which:

FIG. 1 is a schematic diagram illustrating the overall flow of operations through the system of the present invention;
FIG. 2 is a Pathway Data Structure depicting the topology of the pyruvate dehydrogenase reaction in which pyruvate is converted to acetyl-CoA;
FIG. 3 is a block diagram illustrating the flow of information to produce hierarchical descriptions of subcellular and cellular function in which EDS defines an elementary data structure. BDS defines a binary data structure, and PDS defines a pathway data structure;
FIG. 4 depicts a Binary Data Structure;
FIG. 5 illustrates a Binary Data Structure modeling a biophysical process;
FIG. 6 illustrates a Binary Data Structure representing a gene regulatory network;
FIG. 7 is a schematic diagram illustrating the flow of information used to generate structural, finite-element cell models; and
FIG. 8 illustrates a biochemical reaction network.

## DESCRIPTION OF THE PREFERRED EMBODIMENTS

The present invention provides a multidimensional computational tool capable of integrating biological knowledge across all levels of analysis ranging from that of the gene to that of the cell. This is accomplished by a system and method which incorporates at least one database that stores biological information, an interface which displays, links, organizes and modifies that information, and computational engines which operate on the information contained in the database to automatically formulate, solve and analyze computational models of biochemical reaction networks, biophysical mechanisms, and in general dynamic processes at the subcellular and cellular level.

More specifically, the present invention is an interactive computer-implemented system for mathematically modeling biological information from the subcellular to the cellular, tissue, and organ level comprising: (a) at least one database containing biological information which is used to generate at least one data structure having at least one attribute associated therewith; (b) a user interface for interactively viewing and linking together attributes of a plurality of data structures to create at least one hierarchical description of subcellular and cellular function; (c) an equation generation engine operative to generate at least one mathematical equation from at least one hierarchical description; and (d) a computational engine operative on at least one mathematical equation to model dynamic cellular behavior.

The system of the present invention uses computer-implemented tools to link genetic and molecular information to the topological and kinetic properties of biochemical and biophysical processes within cells, to provide functional information on the biochemical and physiological role of gene products. This information is coupled to computational engines that can automatically formulate, interconnect, solve and analyze properties of computational models of genetic, biochemical and biophysical processes within cells. In this way, it is possible to address the functional role played by each molecular/genetic components from which a model is composed, to identify optimal points of therapeutic intervention within these models and to "numerically screen" lead compounds for functional effects on these models.

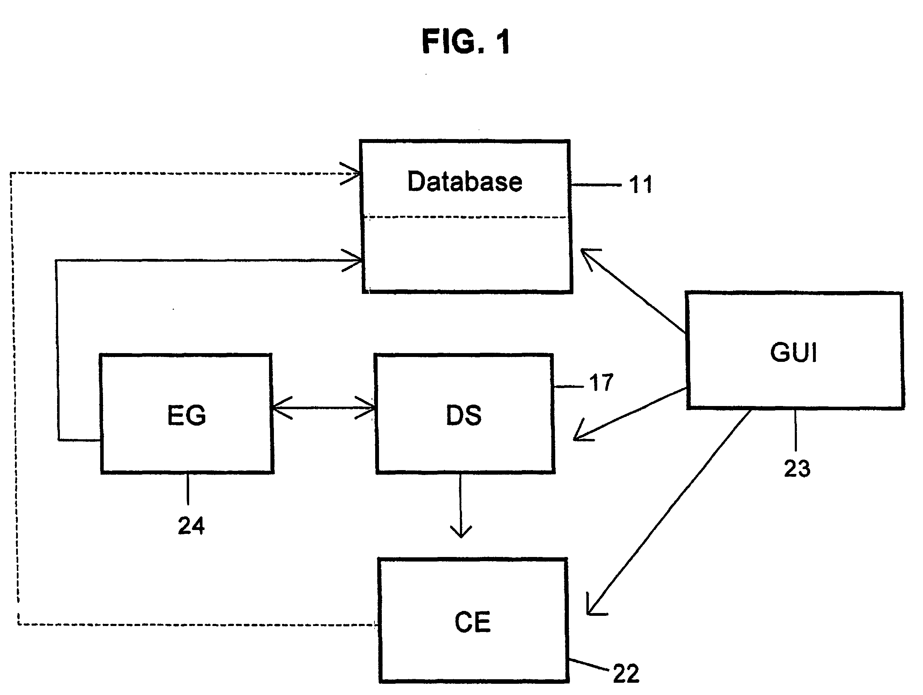

Referring now to the drawings, there is shown in Fig. 1 a schematic diagram illustrating the overall flow of operations of the system of the present invention. Generally stated, the system includes database 11, data structure 17, graphical user interface 23 for interactive contact with the information generated by the system, equation generation engine 24 and computational engine 22.

### Databases

Database 11 encompasses both internal and external databases. External refers to databases designed to store and organize biological information, but which were not designed explicitly to be coupled with the subcellular and cellular modeling, simulation, and analysis tools described herein. Internal refers to databases with a specific structure (to be described in subsequent sections) which are designed explicitly to support the formulation, simulation, and analysis of subcellular and cellular models. Internal and external databases include those containing gene and protein sequences, biochemical and biophysical processes, descriptions of cellular and organ physical structure, experimentally validated models of biochemical and physiological processes, or models previously generated by the system. Database 11 may contain one or any number of the foregoing databases. Any means for accessing and searching external and internal databases may be used in the present invention. Typically these would include: commercial database front-ends with SQL queries, web-based solutions such as Perl scripts and Java-based tools for accessing remote databases, as well as cross-platform software tools available, for example, from Genomica Corp.(Boulder, CO). Pangea Systems, Inc. (Oakland, CA) and NetGenics Inc. (Cleveland, OH). Sun Microsystems, Inc. (Palo Alto, CA) and Microsoft, Inc. (Redmond, WA).

Internal databases include those that have been generated from the data extracted from the external databases as well as data added by users via the graphical user interface. Such data may include experimental data including, for example, new descriptions of biochemical and physiological processes, or it may be data generated as a result of computer modeling by the system. Data generated and stored by the internal databases are manipulated using commercially available object relational or relational database management systems such as Oracle Corp. (Redwood City, CA), Sybase, Inc. (Emeryville, CA), or Informix (Menlo Park, CA), or using markup languages such as SGML or XML, all of which are well known to the skilled artisan. Most importantly, internal databases will store information on the (a) topology; (b) kinetics; and (c) interconnectivity between various genetic and biochemical reaction networks (BRNs) within cells. These will be generically referred to as internal biochemical databases.

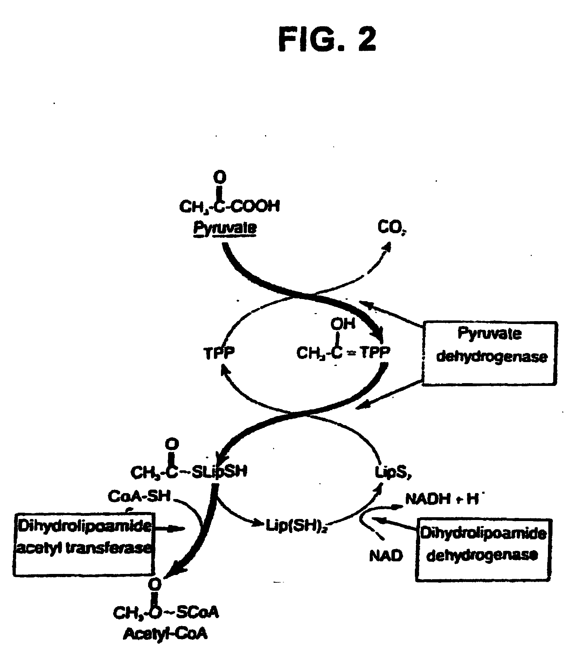

In the context of the present invention, topology refers to the pattern of interactions within a specific genetic or biochemical reaction network; kinetics refers to the reaction rate constants that, in conjunction with the laws of mass action, determine the dynamic behavior of such reaction network processes; and interconnectivity refers to the specific points of coupling between different genetic and biochemical reaction networks within the cell which results in cellular behavior. Thus, the internal biochemical databases store the interconnection topology, including the rate constant associated therewith, for each BRN. By way of example, the BRN for the pyruvate dehydrogenase reaction in which pyruvate is converted to acetyl-CoA is illustrated in FIG. 2. Information on this BRN which would be stored in the internal biochemical databases includes each of the intermediates involved in the reaction, the enzymes involved in determining the rate at which the intermediates are formed (along with lists of co-factors influencing the reaction rate such as pH, temperature, and the like) and the reaction pathways connecting these intermediates. More than one BRN may be linked together to provide a more complex representation of subcellular and cellular behavior.

The internal biochemical databases will store genetic and biochemical reaction network data in a way that makes possible the hierarchical construction of mathematical and computational models of these networks from their underlying components. Once they are formulated. each genetic and biochemical entity within the internal biochemical database may store as attributes a group of symbolic equations and numerical subroutines associated therewith via equation generation engine 24 which allow the user to simulate and view functional behavior of this entity (based on the genetic/biochemical properties of interest) by way of graphical user interface 23, and computational engine 22. In this way, the system will make it possible to link genetic and molecular information to functional information regarding cellular and subcellular processes.

A number of databases are presently available or are currently being developed, *see e.g.,* Popel et al., The Microcirculation Physiome Project, 26 *Annals of Biomedical Engineering* 911-913 (1998). These databases can be created and organized by known software tools which help users build and organize databases such as, for example, those available from Oracle Corp. (Redwood City, CA). Software tools for designing and viewing interactive graphical representations via graphical user interface 23 of these databases are also well known and readily available.

The internal databases will also represent and store information regarding biophysical processes with cells. These databases will be referred to as internal biophysical databases. These databases contain information on the physical properties of biological processes that are required to formulate mathematical and computational models of these processes, as for example, ion channels and currents, membrane transport systems such as pumps and exchangers, membrane receptors and signal transduction pathways for a given cellular process. Once formulated, each physical property may store as attributes a group of symbolic equations and numerical subroutines associated therewith which allow the user to simulate and view cell function (based on the biophysical properties of interest) via graphical user interface 23, equation generation engine 24 and computational engine 22.

Internal databases also contain information on the physical structure and spatial relationship between various organelles within a given cell. These databases will be referred to as internal structural databases. Typically, this information is in the form of three-dimensional image data obtained from different modalities *(e.g.* electron micrograph serial sections, confocal serial sections, two-photon laser scanning serial sections, magnetic resonance images, positron emission tomography images and the like. Optionally, the three-dimensional image data may be further transformed into structural finite-element models of cells describing cell shape and spatial placement of organelles via an optional computational modeling engine which will be discussed in greater detail below. These structural cell models generated from the three-dimensional data are also stored in the structural databases. As with other databases, the structural databases may be publicly available or it may consist of a novel or proprietary database. The structural databases thus contain information on anatomical subcellular and cellular structure for a cell of interest which, in conjunction with the molecular, biochemical and biophysical databases, provides the data necessary to produce a complete model of cellular and subcellular function. But certainly any type of data useful to develop models of subcellular and cellular function is within the scope of the present invention.

By way of example, the precise geometry of and the spatial relationship between cardiac T-tubules and their associated L-type calcium ("Ca") channels and ryanodine-sensitive Ca release channels in the sarcoplasmic reticulum membrane provides information on the properties of calcium-induced calcium release, and therefore mechanical force generation in cardiac muscle cells. Likewise, information about the physical location of Ca-channels and Ca-modulated potassium channels in auditory hair cells provides information about the electrical tuning of these cells or knowledge of the spatial location of subcellular processes in specific cell organelles, e.g. mitochondrial respiration, provides the information necessary for a complete and accurate model of the entire cell. Of course, the foregoing examples are non-exhaustive, and any information on physical structure and spatial relationships therein is well within the scope of the present invention.

External databases used in the present invention may be accessible through known commercial channels or. for example, through the system of global information exchange referred to as the World Wide Web. Typically, these databases contain gene sequence, protein sequence and three dimensional structural data on each constituent of a biochemical reaction network within a given cell, but certainly any type of data useful to develop models of sub-cellular and cellular function is within the scope of the present invention. External databases such as those on the World Wide Web are becoming increasingly standardized so that access to a variety of diverse databases is possible in a single application. *See e.g.*, Markowitz et al., Characterizing Heterogeneous Molecular Biology Database Systems, 2 *J Comput. Biol.* 547-556 (1995). Advantageously, the system of the present invention can access and immediately use the data from the external databases, or alternatively, the system may transfer the information from these databases into another database (not identified) in the system for later use.

### Data structures

The information in database 11 is organized into and stored as at least one data structure which is used to construct a specific model of cellular or subcellular process. Preferably, the data structure comprises either a group of hierarchical description of subcellular and cellular function 17, or alternatively, anatomical data structures describing the physical organization and structure of biological cells.

Data structure refers to a group of interdependent data in which the specific cause-and-effect relationship between the data are not defined. Thus, a data structure would indicate that data are related, not how they are related. Typically, data structures are generated by means of the graphical user interface 23 and the information available in the database 11. Graphical user interfaces and databases can in turn be developed using, for example, software tools available from Microsoft (Redmond, WA) or Oracle Corporation (Redwood City, CA).

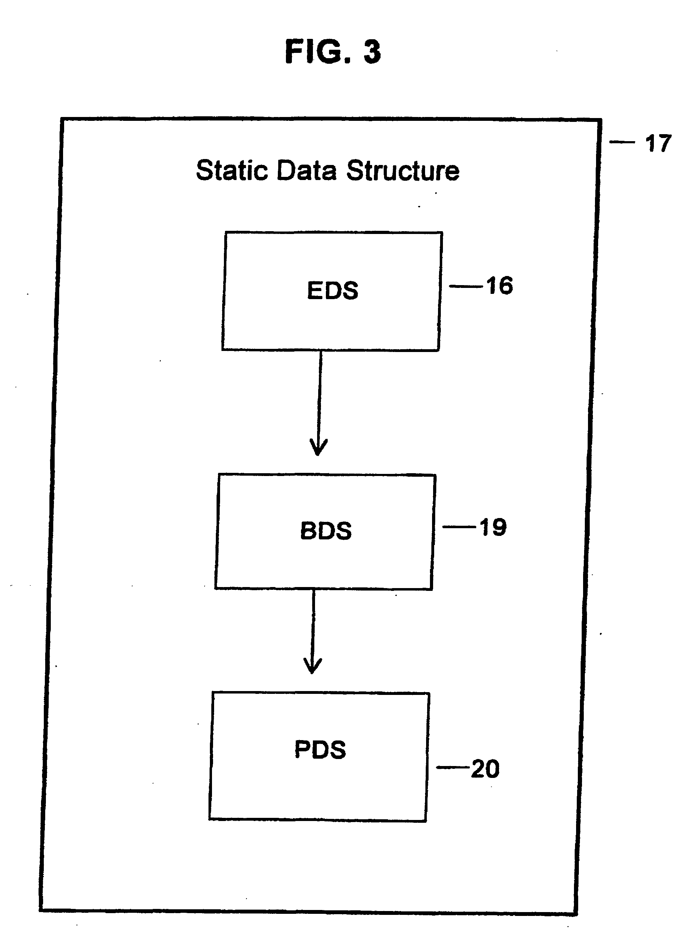

Referring to FIG. 3. data structure 17 comprises elementary data structure 16. binary data structure 19 and pathway data structure 2 with the binary 19 and pathway 20 data structure being formed from lower level data structures. The lowest level data structure is the elementary data structure ("EDS") 17. Each EDS 17 may comprise either a protein i.e., an entity coded by a gene, or a variable. As used herein, a variable refers to anything other than a gene, which defines interdependencies in cell processes as for example, elements or ions important to cell function such as $K^+$, $Na^+$, $Ca^+$, $H^+$, organic or inorganic compounds such as ATP, ADP, $P_i$, or any abstracted quantity describing the state of a biochemical or biophysical process, and which relates to cellular, subcellular, molecular, or genetic function. EDS's may also comprise state variables, the set of parameters needed to calculate the bahavior of the system at a point in time, relating to the models generated by the system.

In accordance with the present invention, each EDS is associated with an extensive set of attributes which describes the EDS. For example, attributes associated with a protein might describe the organism in which the protein is found, the specific cell in which the protein is found, the specific gene coding for the protein, the sequence of the gene coding for the gene and so forth. The attributes describing each EDS are defined and hierarchically arranged by means of the graphical user interface 23.

These hierarchical description attributes thus comprise a grouping of pointers to specific portions in database 11 in which specific information associated with each attribute is found. By way of example, the attributes associated with a given protein could be arranged as Organism:Cell:Gene:State:Sequence:Structure:Location:Model. In this instance, the attribute "Organism" is a pointer to the appropriate gene database in which a gene which codes for the protein exists. The attribute "Cell" points to the specific cell type within that database in which the gene is expressed. The attribute "Gene" is a pointer to the specific gene in the database. The attribute "State" identifies the state of the Organism:Cell:Gene triplet and may be anything that might effect expression of the protein such as an age-related parameter, the presence of a particular disease in the organism, a particular time in the progression of a disease, or the like. Therefore, the attribute "State" is a pointer identifying which particular subset of the Organism:Cell:Gene database to search. The attribute "Sequence" is a pointer to sequence data in the structure of the gene coding for the protein. The attribute "Structure" is a pointer to the three-dimensional structure of the protein coded by that gene, if known. The attribute "Model" is a pointer to a database in which functional models of the protein coded by that gene are stored. Although reference has been made to protein-related attributes, any information regarding biological entities is within the scope of the present invention.

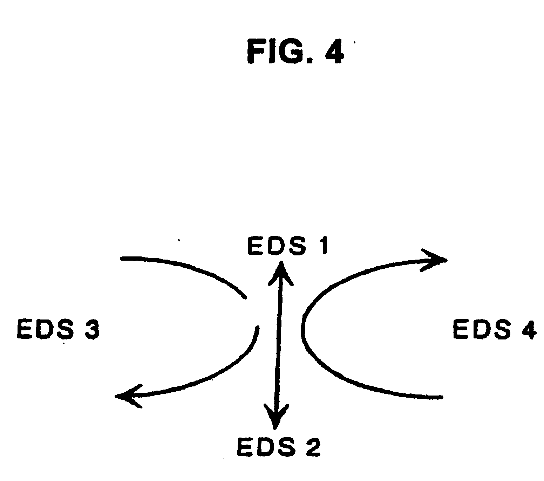

Binary data structure ("BDS") 19 is formed as a composition of more than one EDS. As more specifically illustrated in FIG. 4, BDS 19 comprises separate EDS's with arcs denoting the transitions between these EDS's. In this example, EDS 1 represents the elementary data structure corresponding to state 1 of the binary relationship, EDS 2 represents an elementary data structure corresponding to state 2, and EDS 3 and EDS 4 are elementary data structures determining the forward and backward transition rates, respectively, of the reaction between state I and state 2. This binary representation is also known as a state transition diagram. Binary data structures are generated from knowledge of biophysical and biochemical pathways within cells. They may be derived from interrogation of existing biological databases, or may be generated using graphical user interface 23 from proprietary experimental data. Thus BDS's are the first level data structures at which information on the topology and kinetics of biological reaction networks are represented.

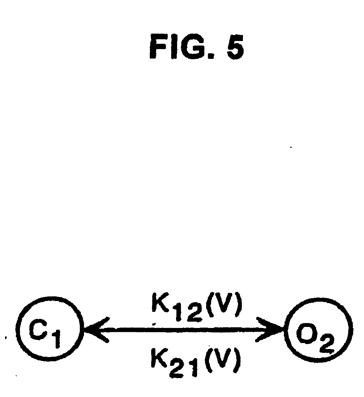

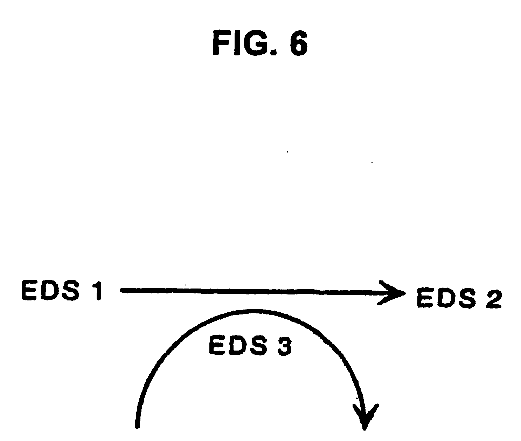

The binary relationship illustrated in FIG. 4 has many analogues in biological systems. For example, the binary relationship may represent transitions between two intermediates within the complex biochemical network shown in FIG 2. In this instance, EDS1 could represent pyruvate (a variable), EDS2 could represent Acetyl-CoA (a variable), EDS 3 could represent the catalytic enzyme pyruvate dehydrogenase (a protein), and EDS 4 could represent the substrate NAD (a variable). Alternatively, the binary data structure could represent a simple two-state closed-open model of a cardiac ion channel, thus modeling a biophysical process as shown in FIG. 5. In this instance, EDS 1 corresponds the closed state of an ion channel (a variable), EDS 2 corresponds to the open state of the ion channel (a variable), and EDS 3 and 4 would be identical and equal to membrane potential V (variables). Because this is a data structure, the functional dependence of the transition rate constants K12 and K21 on quantities such as temperature, pH, membrane potential, and in general variables and/or proteins as defined previously, on membrane potential is not specified, only the fact that a dependence exists is specified. As another example, a binary representative of a gene regulatory network is shown in FIG. 6. Here, EDS 1 represents an RNA polymerase (protein), EDS 2 represents a closed RNA polymerase complex (variable), and EDS 3 represents a promoter (protein). As previously discussed, the data structures do not define the dependence of the transition rates on the underlying elementary data structures. Instead, they only indicate that such a dependence exists.

BDS 19 is also associated with a number of attribute lists. For example, the BDS in FIG. 4 may be represented by the list Input:Output:Frate:Brate wherein the attribute "Input" is associated with EDS1, the attribute "Output" is associated with EDS2, the attribute "Frate" is associated with EDS 3 and describes the forward transition rate, and the attribute "Brate" is associated with EDS 4 and describes the backward transition rate. As with the EDS's, graphical user interface 23, or an interface into existing biological database 11, would be used to generate the attribute lists.

BDS 19 retains the attributes of each EDS which it comprises. Thus, the attribute lists defining BSD 19 would have multiple attributes reflecting the group of attributes associated with each EDS. Therefore, a BSD may have distinct attributes of the Organism:Cell:Gene:State:Sequence:Structure:Location:Model attribute list discussed previously, but would not contain the single "Gene", "Sequence" or "Structure" attribute each is associated with a single EDS.

Pathway data structure ("PDS") 20 represents the highest level of data structure and is generated as the composition of more than one BDS. An example of a PDS is the pyruvate dehydrogenase reaction depicted in FIG. 2. Thus, PDS 20 represents a more complex state transition diagram which retains the attributes of the EDS's and BDS's present in the pathway.

PDS 20 is also associated with a number of attribute lists. Because PDS 20 retains the attributes of its constituents, the attribute list Organism:Cell: Gene:State:Sequence:Structure:Location:Model described above may be applied to PDS 20. The modeling tools used to organize the databases and generate the EDS's, BDS's and their associated data may be used to generate the PDS's.

In accordance with the present invention, any biochemical reaction and physiological process can be arranged into a state transition diagram and its associated attribute list. Typically, the data associated with the data structures is stored in database 11 or is generated by a user either prior to or at the time of model construction. Advantageously, the system model is configured so that a user can interact with graphical user interface 23 to retrieve any of the data associated with or generated by the data structures and their associated linked lists.

Data structure 17 may also comprise at least one anatomical data structure describing the physical organization and structure of biological cells. These data structures may be in the form of sets of three-dimensional image data from structural database as previously discussed. Alternatively, a computational engine, known as a geometry modeling engine may transform the three-dimensional image data into structural finite-element models of cells describing cell shape and spatial placement of organelles therewithin. Geometry modeling engines such as EnSight (available from CEI, Inc., Research Triangle Park, NC) and FIDAP (available from Fluent Inc., Lebanon, NH) are well known and readily available. Each of the three-dimensional image data or the finite element cell models may be stored in the system for later use or generated as necessary.

Like the other data structures, the three-dimensional image data and the structural finite element cell models have specific attributes. Typically, these attributes are in the form Organsim:Cell:Organelle:Modality:ImageFormat, wherein the attributes "Organism" and "Cell" are as discussed above. "Organelle" is a pointer to that part of the anatomical database on structure of the specific organelle, "Modality" defines the type of anatomical data (such as a model derived from the three-dimensional image data or the three-dimensional image data itself), and "ImageFormat" defines the structure of the anatomical data.

The geometry of the cells and organelles may be viewed on graphical user interface 23 so that the shape of and spatial relationship between cells and organelles is observed.

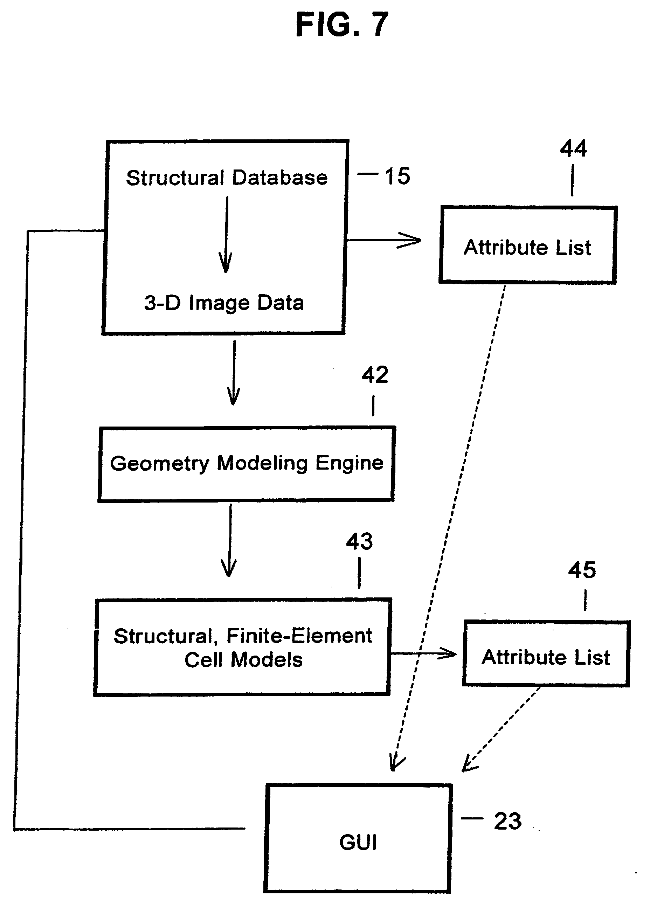

As more specifically illustrated in FIG. 7, three-dimensional image data from structural database is defined by attribute list 44. This three-dimensional image data may be further transformed by geometry modeling engine 42 into structural finite-element cell model 43 which may be used to create additional list 45. During the creation of a subsequent model, a user would have access to any of the three-dimensional image data from structural database 15, structural finite-element cell model 43, or attribute list 44 or 45. As such, the anatomical data structure may be specifically tailored to subsequent model use.

### Computational/Equation Generation Engines

Generally stated, computational engines transform data structure 17 into mathematical models of biochemical, physiological and structural cellular and subcellular processes. Advantageously, the interconnection topology specified in each data structure permits the computational engine to automatically generate these biological models by applying the laws of mass action.

Computational engine 22 generically refers to an equation generation engine for generating symbolic models of biological processes as well as an engine for generating computational models based upon the symbolic models. Equation generation engines 24 such as those which are a part of commercially available software tools such as MathML, Mathematica Maple are well suited to the practice of the present invention. The equation generation engine 24 automatically transforms each data structure into at least one system of equations describing a specific biologic process. This system of equations is referred to as a symbolic model. These symbolic models may be stored in the system for later use in modeling the same biologic process, or alternatively, the models may be coupled with other symbolic models generated by the system to model different biologic processes. Advantageously, any number of symbolic models may be coupled together to produce a multidimensional model of a more than one cellular or subcellular process. In this way, many different cellular or subcellular processes may be incorporated into a single model. Thus, the system of the present invention permits the modeling of the entire cell with each of its respective subcellular processes. Therefore, the present invention differs from the piecemeal one-dimensional approach used to study cellular processes reported in the prior art.

Computational engine 22 generates a computational model reflective of the biological process defined by the symbolic model. A computational model refers to a software procedure for numerical simulation of the symbolic model. As previously noted, computational models are software procedures for numerical simulation of the behavior of the symbolic model. Typical tools used to generate numerical simulations include those available from IMSL (International Mathematical and Statistical Library); NAG (Numerical Algorithm Group); and MATLAB (Mathematical Laboratory) and the like.

Optionally, the symbolic models may also be translated into computer code such as Fortran, C++, by conventional means readily available in the prior art for use in generating computation models. Advantageously, typeset equations expressed in markup languages such as TeX, LaTeX or HTML can be automatically derived from the symbolic models which tremendously simplifies the process of model documentation. Moreover, critical components of computational models for example, Jacobian matrices that are used by certain numerical integration algorithms can be derived in an automated fashion from the symbolic models.

Accordingly, the symbolic and computational models therefore define the time rate of change of the concentration of reaction intermediates, or of other state variables that effect cellular and subcellular processes.

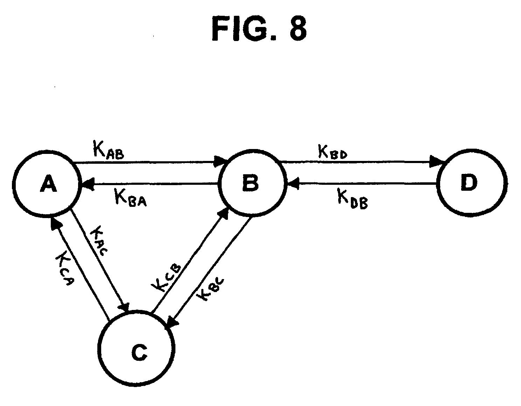

By way of example. consider the biochemical pathway shown in FIG. 8. Let A, B, C, and D represent elementary data structures defining the pathway wherein "i" or "j" are generic representations for the various states such as A, B, C, or D ($K_{ij} \approx K_{AB}$ or $K_{CA}$ or ...), and $K_{ij}$ represents the transition rate constant between states i and j that are defined by the various Frate and Brate pointers. Applying the laws of mass action will yield the following system of ordinary differential equations describing the dynamics of this system.

$$dA/dt = -A(K_{AB} + K_{AC}) + BK_{BA} + CK_{CA}$$

$$dB/dt = AK_{AB} - B(K_{BA} + K_{BC} + K_{BD}) + CK_{CB} + DK_{DB}$$

$$dC/dt = AK_{AC} + BK_{BC} - C(K_{CA} + K_{CB})$$

$$dD/dt = BK_{BD} - DK_{DB}$$

Since these equations are completely defined by knowledge of the connectivity of the network, and knowledge of the various transition rate constants, and since these quantities are all stored in the databases, the equations may be generated automatically on computer. They may also be integrated in time, or be analyzed using the numerical methods described herein.

As previously indicated, equation generation engine 24 automatically generates symbolic models in the form of coupled systems of differential equations from the information contained in the data structures. The models so generated will retain the attributes of every component of the data structures used to generate the model. For example, the attributes Organism:Cell:State:Location:ModelType would contain the attributes "Organism", "Cell", "State", and "Location" as previously discussed, with the attribute "ModelType" reflective of the model as symbolic or computational. In this instance, subcategories of models. such as stochastic or a system of ordinary differential equations, could also be defined. By using coupled differential equations, an enormous amount of information can be incorporated into each model allowing for the study and simulation of very complex biochemical reaction networks. Thus, the system of the present invention also accommodates the rapid acquisition and refinement of experimental data. As experimental data become available, hierarchical descriptions containing the interconnection topology can be generated which provides the basis to automatically generate a set of differential equations that can be numerically solved. This information can be coupled with known or new information on cellular or subcellular processes to produce fully accurate models of complex biological processes.

The models generated by the system may be further transformed into textual or graphical representations by use of graphical user interface 23. Optionally, the models may also be analyzed using techniques from nonlinear systems theory. For example, public domain tools accessible from the WWW such as AUTO and XPP can be used to perform analyses of the parameter dependence and asymptotic behaviors of biological models. This permits the calculation of qualitative behaviors of complex models as key model parameters are changed.

## Graphical User Interface

Graphical user interface 23 provides a user with input to and output from information in the system. More specifically, graphical user interface 23 may be used to: (1) draw genetic and biochemical pathway diagrams, and to enter functions specifying rate constants in these reaction pathways, for storage in database 11 or for symbolic and computational modeling; (2) interconnect EDS, BDS, and PDS data structures in order to compose hierarchical models of biological systems; (3) construct and manipulate biophysical and structural models; (4) display and interact with previously developed genetic, biochemical, biophysical, and structural models; and (5) control formulation and solution of computational and symbolic models, and to view simulation output.

Graphical user interface 23 can be customized for a particular application. Typically, interface elements such as video monitors, touchscreens, keyboards, a mouse, printers and the like may be used.

## Creation of a Model

In accordance with the present invention a model may be created to study any type of cellular or subcellular biologic information as, for example, the function of a gene, a specific biological process, the behavior of a target protein in the presence of a particular drug, or the like. Based on the problem to be solved, the user will select the information from the database that will serve as the building blocks for developing the model. For example, a user may wish to predict the quantity of certain intermediates in the pyruvate dehydrogenate reaction in a specific cell type both in health and disease. In this instance, a model would be generated based upon the structural elements of the cell together with the biochemical and biophysical processes and their associated interconnection topologies.

In this example, a user would select the appropriate anatomical structure form the anatomical database. This may be selected from raw image data, a model generated by the user from raw image data stored in the database or, alternatively, a structural model previously stored in the structural database. If, for example, the user elects to build a model from raw image data stored in the database, the user could select and manipulate a single raw image data set from the database to display individual images from the selected image set; render and display and manipulate three-dimensional representations of the cell being reconstructed from image serial sections, process two-dimensional and three-dimensional images to enhance object boundaries, automatically segment or draw two-dimensional and three-dimensional images into discrete objects and bounding membranes; automatically define computational grids with two-dimensional and three-dimensional objects; and generate geometric models of these two and three-dimensional objects.

Once the two and three dimensional models have been constructed or accessed from the database, these models can be displayed on the display monitor. In general, the user will be presented with a palette of icons that can be browsed, where each icon represents some binary or pathway data structure, such as a biochemical or biophysical mechanism previously defined and stored in the system. The user would interact with this graphical display by use of a mouse. The user can add these components to the structural model by selecting icons and dragging them to the point of insertion in the model.

The user may view information regarding the biochemical/biophysical mechanism inserted into the model by clicking on the representation of that mechanism. For example, clicking on the icon for the pyruvate dehydrogenase reaction will trigger a display of the pathway illustrated in FIG. 2 on the display monitor. The user can then query the system for information associated with the intermediates of these reactions. Clicking on, for example, pyruvate dehydrogenase will initiate a pop-up display of all of the attributes describing pyruvate dehydrogenase that may be examined. The user will select from one of these attributes. Advantageously, due to the linked attribute list (*e.g.* Organisim:Cell: Gene:State:Sequence: Structure:Location:Model), used by the system, this selection action will initiate a query and display of information to the appropriate database, for example, a display of the gene sequence of pyruvate dehydrogenase. All of the elements of the attribute list associated with pyruvate dehydrogenase could be displayed in this manner. Thus, the simple act of clicking on pyruvate dehydrogenase retrieves for the user all information on pyruvate dehydrogenase stored in the system and makes it available to facilitate modeling. This configuration permits a user to interact with graphical user interface 23 to retrieve any of the information associated with or generated by the system. In this way, the user is presented with a complete representation of specific biological processes.

If desired, the user can invoke an equation generation engine to generate a symbolic set of coupled differential equations defining the model. These equations could be saved as part of a documentation of the model and/or they may be input into translators that would map them into computer instructions in the desired programming language. This source code can then be linked with a computational engine to produce executable code for modeling the cell. Preferably, this executable code may be stored in the system for future use.

## Linking Models

Several models may be linked together. For example, a number of different biochemical or biophysical mechanisms may be inserted into a single structural model. In this instance, several models would be merged into a single model by an interface which would effectuate the flow of information between the respective models. For example, the outputs or intermediates in a biochemical reaction network (describing a PDS) such as described in FIG. 2, may act directly or indirectly to modulate the function of another process, such as the BDS representing an ion channel model of FIG. 5. A specific case may be the output variable of adenosine triphosphate (ATP) of glycolytic biochemical reaction networks and its modulating action of ATP-sensitive membrane potassium channels.

## Display of Model Results

Output data from each simulation, as well as the underlying data, may be displayed on the graphical user interface. A user can modify the data from each simulation as well as the underlying information which the data represents. The user may also customize the physical appearance of the graphics or textual appearance of the output data. By way of illustration, the user can double-click on a compartment of the model, and would be presented with a list of state variables used. The user could select a variable and display that variable on a graph drawn in a separate window. Optionally, the user could modify the underlying state variable and generate a new model. Alternatively, the user could select "global" variables, that is, those state variables defined everywhere within a model and display the global variable using a color coding scheme over the entire model domain.

## Model Uses

The model can be used to store and search all existing biological information (i.e., genetic, biochemical, biophysical and anatomical) on a given biological process at the subcellular, cellular or multicellular level. As such, the model may be used to integrate knowledge across all biological systems. The model thus provides a means for collecting and synthesizing biological information into a format by which function within a biological system may be analyzed. For example, the function of a particular gene could be ascertained by invoking the model to determine the sequence of the gene of interest and identify homologous genes and BRN's in which the homologous gene participates. Based on the BRN's, the dynamic behavior of the homologous genes could be modeled, providing quantitative insight into the possible functional role of the gene of interest. Thus, the model could provide not only homology searches based on linear sequence analysis, but also functional search capabilities based on the similarity of the BRN's in which a gene participates.

In addition, the model may be used in drug discovery, as for example, to analyze the behavior of molecular targets in the presence of a particular drug. Computational models of drug/gene action would be generated and incorporated into models of physiological function in accordance with the present invention. These multi-dimensional models could then be used to screen candidate compounds.

## Computer System

The present invention may be implemented on any computer architecture in any configuration such as multi-tiered or clustered services or a client-server paradigm. Certainly, the type of computer system will depend on the complexity of the model(s) and the choice of an appropriate system is readily available to a skilled artisan. Typically, the components of such a computer system would include a central processing unit, RAM, ROM, I/O Adapter, data storage space, and a graphical user interface having a keyboard, mouse and speakers attached thereto.

The following examples are presented to provide a more complete understanding of the invention. The specific techniques, conditions, materials, proportions and reported data set forth to illustrate the principles and practice of the invention are exemplary and should not be construed as limiting the scope of the invention.

**Example 1**

**Example 2**

This is an example of a CellML description of the basic FitzHugh-Nagumo model. For purposes of this model it is treated as an ion current.

This model contains two differential equations :

$$du/dt = (u - u^3/3 - v) / e \text{ and } dv/dt = e * (u + b - g v)$$

Where b, g, and e are treated as constants.

<CELLMODEL>

 <VERBOSENAME>Simple Example of a cell model with a single FitzHugh-Nagumo element</VERBOSENAME>

 <NAME>FitzHugh-Nagumo Cell</NAME>

A <DRAW> tag is used by the program to describe how the object is represented visually in the cell model. -->

 <DRAW>

  <DRAWSIZE>8000,8000</DRAWSIZE>

  <POSITION>1000,1000</POSITION>

  <BACKCOLOR>65280</BACKCOLOR>

  <EDGECOLOR>255</EDGECOLOR>

 </DRAW>

The ENVIRONMENT tag is used to define all of the components (chemical species, variables, etc.) within the scope of an element. -->

 <ENVIRONMENT>

CONSTANT tags are used to contain information about the value of parameters used in this model

```
<CONSTANT>
    <NAME>b</NAME>
    <VALUE>1.0</VALUE>
</CONSTANT>

<CONSTANT>
    <NAME>e</NAME>
    <VALUE>0.04</VALUE>
</CONSTANT>

<CONSTANT>
    <NAME>g</NAME>
    <VALUE>0.5</VALUE>
</CONSTANT>
```

VARIABLE tags are similar CONSTANT tags except that the values can change during the execution of the model. The values given here represent the initial value for the variable

```
<VARIABLE>
        <NAME>t</NAME>
    <VALUE>0.0</VALUE>
    </VARIABLE>


    <VARIABLE>
        <NAME>u</NAME>
    <VALUE>0.0</VALUE>
    </VARIABLE>


    <VARIABLE>
        <NAME>v</NAME>
    <VALUE>0.0</VALUE>
    </VARIABLE>


    </ENVIRONMENT>
```

An IONCURRENT is used to contain the actual model.

```
<IONCURRENT>
<NAME>Ifn</NAME>
<VERBOSENAME>FitzHugh Nagumo Current</VERBOSENAME>


    <DRAW>
    <DRAWSIZE>1000,1000</DRAWSIZE>
    <POSITION>6000,6000</POSITION>
    <BACKCOLOR>32639</BACKCOLOR>
    <EDGECOLOR>8323199</EDGECOLOR>
    </DRAW>
```

The equation for du/dt. The <DERIVATIVE> tag is used to indicate that this needs to be processed as a differential equation

```
<DERIVATIVE>
<reln>
 <eq/>
 <apply>
  <diff/>
  <ci>u</ci>
  <bvar>
   <ci>t</ci>
  </bvar>
 </apply>
 <apply>
  <divide/>
   <mfence>
   <apply>
    <minus/>
    <apply>
     <minus/>
     <ci>u</ci>
      <apply>
       <divide/>
       <apply>
```

```
                    <power/>
                    <ci>u</ci>
                    <cn>3</cn>
                </apply>
                <cn>3</cn>
            </apply>
        </apply>
        <ci>v</ci>
    </apply>
</mfence>
<ci>e</ci>
</apply>
</reln>
</DERIVATIVE>
```

The equation for dv/dt.

```
<DERIVATIVE>
<reln>
 <eq/>
 <apply>
  <diff/>
  <ci>v</ci>
  <bvar>
   <ci>t</ci>
  </bvar>
 </apply>
 <apply>
  <times/>
  <ci>e</ci>
  <mfence>
```

```
<apply>

    <minus/>

    <apply>

        <plus/>

        <ci>u</ci>

        <ci>b</ci>

    </apply>

    <apply>

        <times/>

        <ci>g</ci>

        <ci>v</ci>

    </apply>

    </apply>

    </mfence>

    </apply>

    </reln>

    </DERIVATIVE>

    </IONCURRENT>

</CELLMODEL>
```

## Example 3

This Example describes the XML tags used by InSilicoCell to represent a cell model. For the purposes of InSilicoCell, these tags can be thought of as a description of "CellML" (though we are not the first to use this term...). CellML is a subset of XML that is used to describe a cell model or series of cell models.

CellML uses MathML to model the actual equations that it references.

The tags in CellML are designed to be hierarchical in nature; that is, a given tag is generally used to describe the properties of its parent. For example, a <SIZE> tag can be used to indicate the size of a <CELLMODEL>. When the CellML code is read by the InSilicoCell XML parsing engine a series of "objects" (i.e. class objects in C++ or Java parlance) is creating that has close to a one-to-one correspondence with the original source code.

CellML tags are broken down into several distinct classes, based on their purpose:

- **Basic Elements** are tags that are used to describe a general property such as the name of an object or its size. These are the lowest level elements, and can be used by several different kinds of tags.

- **General Cell Model Elements** are used to represent the general properties of a cell and the biochemical processes that are being model.

- **Specific Cell Model Elements** are similar to "General Cell Model Elements" except that they are used to represent a higher level of abstraction.

- **Drawing Elements** are used to supply information on how a Cell Model is to be displayed visually, and how it interacts with the GUI.

The contents of each CellML document will obey a set of grammar rules defined in the CellML Document Type Definition (DTD).

| TYPE | TAG | DESCRIPTION | SUB-TAG |
|---|---|---|---|
| **Basic Elements** | NAME | The short name of an object | --- |
| | VERBOSENAME | A longer name for the object | --- |
| | VALUE | Tag used to store a single numeric value | --- |
| | CONSTANT | Used to define a fixed parameter | NAME<br>VALUE<br>UNITS |
| | VARIABLE | Used to represent a single state variable. This contains both a value at the current point in time, and at the initial condition. | NAME<br>VALUE<br>UNITS<br>HISTORY |
| | UNITS | The units for a VALUE (e.g. [mm], [g/mol], etc.) | --- |
| | EQUATION | Used to contain a single MathML equation. | RELN (MathML code) |
| | POSITION | The physical position of an object in its parent object. Can be used to define 3D (x,y,z), 2D (x,y) or ID (x) position. | --- |
| | SIZE | The physical size of an object. Can be 3D, 2D, or ID. | --- |
| | DBLINK | Database Linkage. Used to hold a pointer to information on an element in a database. | |
| **General Cell Model Elements** | MODEL | The highest level object, consisting of one or more CELLMODELS. | CELLMODEL |
| | CELLMODEL | A single unit in a model. This tag may contain information about it's location relative to other CELL MODELS | PATHWAY REACTION COMPONENT IONCURRENT REFERENCE |

| TYPE | TAG | DESCRIPTION | SUB-TAG |
|---|---|---|---|
| **General Cell Model Elements** | PATHWAY | Tag that describes a set of reactions, for example where:<br>Reactant $\longleftrightarrow$ Product in multiple steps.<br>(PDS) | REACTION |
| | REACTION | Describes a single elementary reaction.<br>Reactants $\longleftrightarrow$ Products with Forward and Reverse kinetics. (BDS) | COMPONENT<br>KF<br>KR |
| | ENVIRONMENT | Encapsulates all of the components and properties of a CellModel | COMPONENT CONSTANT |
| | COMPONENT | Representation of a single chemical species. This tag can contain information on concentration, formula, and structure. (EDS) | VARIABLE DBLINK |
| | HISTORY | The value of a "property" (e.g. COMPONENT, VARIABLE) as a function of time. | |
| | KF | Forward Reaction kinetics for single REACTION. | COMPONENT EQUATION |
| | KR | Reverse Reaction kinetics for single REACTION. | COMPONENT EQUATION |
| | INTEGRATE | Used to store information about the type of integration to be run. Contains starting and stopping time, time step, and type of integrator to be used. | |
| | PROTOCOL | Description of a time based protocol applied to a Cell Model | VARIABLE |
| | REFERENCE | A bibliographic tag used to describe where this model came from. This will ultimately contain several subtags for elements such as "<author>, "<volume>", "<date>", etc. | |
| **Specific Cell Model Elements** | IONCURRENT | Used to represent an ion current. | GATE COMPONENT |
| | GATE | A Hodgkin-Huxley type gate element. | EQUATION |
| | PROTEIN | Descriptions of a gene product. | |
| | DRUG | Description of drug effect on elementary, binary or pathway data structures or protein or variable. | |

(continued)

| TYPE | TAG | DESCRIPTION | SUB-TAG |
|---|---|---|---|
| **Drawing Elements** | DRAW | Tag encapsulating information needed to draw an object in a window. | SIZE<br>POSITION<br>FORECOLOR<br>BACKCOLOR<br>EDGECOLOR |
| | LOGCOORD | Tag containing information on transforming from physical to logical coordinate system. This can also control the rendering of a 3D object on a 2D screen. | |
| | FORECOLOR | The foreground color of an object | |
| | BACKCOLOR | The background color of an object | |
| | EDGECOLOR | The color of an objects edge | |
| | POSITION | The position of an element in logical drawing space. | |
| | DRAWSIZE | The size of an element in logical drawing space | |

**Example 4**

| InSilicoCell Components | |
|---|---|
| **Component** | **Description** |
| Class Library | The C++ class objects that are used to create a cell model and simulation, and describe its mathematics and chemistry. |
| CellML Definition / DTD | A definition of the mark-up language used to describe the InSilicoCell models. This involves developing the set of tags to use with CellML, and putting together a DTD to formalize the syntax and allow models to be validated by browsers. |
| Parser | The Parser is used to generate "run-time objects" of the cell components based on an XML input file and the InSilicoCell class library. This consists of two components: (1) The raw XML parser that reads the input files and generates the hierarchical tag and text nodes. (2) The "object constructor" which creates and initializes the InSilicoCell objects based on the XML content. |
| Class Converter | Conversion of XML tags to leaner MathML class objects |
| Computation Engines | Systems that are used to integrate cell model over time and evaluate reactions. |
| Component Editor | A form-based GUI that is used to create and initialize the set of chemical components within an "environment" of a cell model. |
| Reaction Editor | A form-based GUI used to graphically create a chemical reaction or pathway. |
| Equation Editor | Used to allow mathematical equations to be entered into models in an algebraic format (as opposed to the native MathML format). |
| Database Linkage | Used to connect InSilicoCell to external database system containing information on cell components. |
| Visual Editor | Allows the user to graphically edit a cell model using features such as drag-and-drop and in-place activation. |
| Data Plotting System | A generic 2D and possibly 3D plotting system. This is a full-featured system giving complete control over the layout, scaling, and visual format of a plot. |
| Dynamic Form System | This system is used to create a dialog form from an XML input file or an InSilicoCell model object. This allows cell models to be edited and manipulated in a very flexible way. |
| Object Serialization | Used to read and write (serialize) object-based cell models in a binary format. This is required to enable releasing proprietary cell models. |
| Output Engines | The output engines are used to take an object based cell model and generate text output in several different formats. Formats being considered include: (1) XML output file. (2) Fortran and/or C equations defining the cell model behavior. (3) Some form of visual presentation of the mathematical equations (HTML/MathML, TeX, rich-text). |
| Java / Web Model Viewer | A tool that allows CellML models to be viewed in a browser. |
| Java-based Model Editor | An InSilicoCell editor designed to run in a Java environment. |
| User Documentation | User manual describing the use and operation of InSilicoCell |
| Online Help System | Online version of user manual and integration of this into InSilicoCell program. |

**EP 1 171 841 B1**

Having thus described the invention in rather full detail, it will be understood that such detail need not be strictly adhered to but that various changes and modifications may suggest themselves to one skilled in the art, all falling within the scope of the present invention as defined by subjoined claims.

**Claims**

1. An interactive computer-implemented system for constructing computer-executable models of the dynamic behaviour of biological processes based on several types of biological information at the cellular and subcellular level, comprising:

   a) at least one database (11) comprising an internal and external database containing said several types of biological information which is used to generate at least one data structure (17), wherein the data structure is selected from the group consisting of an elementary data structure, a binary data structure, a pathway data structure, or a combination thereof, each of the data structures of said group being associated with at least one attribute;
   b) a graphical user interface (23) for interactively viewing and arranging data structure attributes to generate a hierarchical description of a biological process;
   c) an equation generation engine (24) operative on the data structure to generate a system of differential equations describing a symbolic model of the biological process; and
   d) a computational engine (22) operative on the system of differential equations to generate a computer-executable model of the dynamic behaviour of the biological process.

2. An interactive computer-implemented system as recited by claim 1, wherein the symbolic model may be stored in the system for subsequent modeling.

3. An interactive computer-implemented system as recited by claim 1, wherein at least two symbolic models are generated by the equation generation engine and coupled together by the system to produce a multidimensional model of a cellular or subcellular process.

4. An interactive computer-implemented system as recited by claims 1 or 3, wherein the symbolic model comprises at least two differential equations.

5. An interactive computer-implemented system as recited by claim 1, wherein the data structure is an anatomical data structure.

6. An interactive computer-implemented system as recited by claim 5, wherein the anatomical data structure is further modified to form at least one structural cell model describing cell shape and special placement of organelles therewithin.

7. An interactive computer-implemented system as recited by claim 1, wherein the binary and pathway data structures are arranged as state transition diagrams.

8. An interactive computer-implemented system as recited by claims 1 and 4, wherein at least one hierarchical description of subcellular function is further modified to generate a computer-executable model.

9. An interactive computer-implemented system as recited by claim 1, wherein the database is selected from the group consisting of gene sequence data, protein sequence data, anatomical data, biochemical data, biophysical data, or a mathematical model of the foregoing.

10. An interactive computer-implemented system as recited by claim 1, wherein the binary data structure is a composition of at least two elementary data structures having at least one transition there between.

11. An interactive computer-implemented system as recited by claim 1, wherein the binary data structure is a composition of at least two elementary data structures having at least one rate constant associated therewith.

12. A computer-implemeted method for constructing computer-executable models of the dynamic behaviour of biological processes based on several types of biological information at the cellular and subcellular level, comprising:

a) accessing at least one internal and external database containing said several types of biological information;

b) generating at least one data structure selected from the group consisting of an elementary data structure, a binary data structure and a pathway data structure or a combination thereof, wherein each of the data structures of said group has at least one attribute associated therewith;

c) generating a hierarchical description of a biological process, by interactively viewing and arranging data structure attributes;

d) utilizing an equation generation engine to generate a system of differential equations describing a symbolic model of the biological process; and

e) utilizing a computational engine to transform the system of mathematical equations to a computer-executable model of the dynamic behaviour of the biological process.

13. A computer-implemented method as recited by claim 12, further comprising the step of interactively viewing at least one of the data structures, attributes or hierarchical descriptions of a biological system.

14. A computer-implemented method as recited by claim 12, wherein the symbolic models may be stored in the system for subsequent modeling.

15. A computer-implemented method as recited by claim 12, wherein at least two symbolic models are generated by the equation generation engine and coupled together by the system to produce a multidimensional model of a cellular or subcellular process.

16. A computer executable model of the dynamic behaviour of a biological process as constructed with the system of claim 1, comprising a model of dynamic cellular behavior mathematically generated from at least one hierarchical description of the biological process, wherein the hierarchical description is generated from a plurality of data structures wherein each data structure is generated from at least one database comprising an internal and external database containing biological information, and wherein each data structure has at least one attribute associated therewith and is selected from the group consisting of an elementary data structure, a binary data structure, a pathway data structure, or a combination thereof.

17. A computer executable model as recited by claim 16, wherein the hierarchical description is further transformed into a symbolic model of the biological process.

18. A computer executable model as recited by claim 17, further comprising computer executable code generated from the symbolic model.

19. A computer program product for carerying out the method of claim 12, when run or a computer, comprising:

a) a data storage structure comprising at least one database containing biological information wherein the biological information is arranged into at least one data structure having an attribute associated therewith, and wherein the data structure is selected from the group consisting of an elementary data structure, a binary data structure, a pathway data structure, or a combination thereof;

b) code means for operating on the data structure to generate a system of differential equations describing a symbolic model of a biological process, and

c) code means for operating on the system of differential equations to generate a computer-executable model of the dynamic behaviour of the biological process.

20. A method of storing and searching biological information in the system as claimed in claim 1, comprising the steps of

a) using the computer-implemented system to access information from at least one database comprising an internal and external database containing biological information;

b) creating at least one data structure having an attribute associated therewith from the information in the database, wherein the data structure is selected from the group consisting of an elementary data structure, a binary data structure, a pathway data structure, or a combination thereof; and

c) arranging the data structure to generate a hierarchical description of biological information.

21. A method as recited by claim 20 further comprising the step of storing the data structure in the system.

22. A method as recited by claim 20 further comprising use of a graphical user interface for interacting with the data structure.

**Patentansprüche**

1. Interaktives computer-implementiertes System zum Konstruieren computer-ausführbarer Modelle des dynamischen Verhaltens von biologischen Prozessen basierend auf mehreren Typen biologischer Information auf zellulärem und subzellulärem Niveau, umfassend:

   a) wenigstens eine Datenbank (11) umfassend eine interne und externe Datenbank, welche die mehreren Typen biologischer Information enthält, welche verwendet wird, um wenigstens eine Datenstruktur (17) zu erzeugen, wobei die Datenstruktur aus der Gruppe ausgewählt ist, die aus einer elementaren Datenstruktur, einer binären Datenstruktur, einer Weg-Datenstruktur oder einer Kombination von diesen besteht, wobei jede der Datenstrukturen der Gruppe mit wenigstens einem Attribut assoziiert ist;
   b) eine graphische Benutzerschnittstelle (23) zum interaktiven Betrachten und Bearbeiten von Datenstrukturattributen, um eine hierarchische Beschreibung eines biologischen Prozesses zu erzeugen;
   c) eine Gleichungserzeugungs-Maschine (24), die auf der Datenstruktur arbeitet, um ein System von Differentialgleichungen zu erzeugen, welches ein symbolisches Modell des biologischen Prozesses beschreibt; und
   d) eine Rechenmaschine (22), die auf dem System von Differentialgleichungen arbeitet, um ein computer-ausführbares Modell des dynamischen Verhaltens des biologischen Prozesses zu erzeugen.

2. Interaktives computer-implementiertes System gemäß Anspruch 1, bei welchem das symbolische Modell in dem System für ein anschließendes Modellieren gespeichert werden kann.

3. Interaktives computer-implementiertes System gemäß Anspruch 1, bei welchem wenigstens zwei symbolische Modelle von der Gleichungserzeugungs-Maschine erzeugt werden und von dem System verkoppelt werden, um ein multi-dimensionales Modell eines zellulären oder subzellulären Prozesses herzustellen.

4. Interaktives computer-implementiertes System gemäß Anspruch 1 oder 3, bei welchem das symbolische Modell wenigstens zwei Differentialgleichungen umfaßt.

5. Interaktives computer-implementiertes System gemäß Anspruch 1, bei welchem die Datenstruktur eine anatomische Datenstruktur ist.

6. Interaktives computer-implementiertes System gemäß Anspruch 5, bei welchem die anatomische Datenstruktur weiter modifiziert wird, um wenigstens ein strukturelles Zellenmodell zu bilden, das die Zellenform und spezielle Anordnung von Organellen in dieser beschreibt.

7. Interaktives computer-implementiertes System gemäß Anspruch 1, bei welchem die binären und Weg-Datenstrukturen als Zustandsübergangsdiagramme eingerichtet sind.

8. Interaktives computer-implementiertes System gemäß Ansprüchen 1 und 4, bei welchem wenigstens eine hierarchische Beschreibung einer subzellulären Funktion weiter modifiziert wird, um ein computer-ausführbares Modell zu erzeugen.

9. Interaktives computer-implementiertes System gemäß Anspruch 1, bei welchem die Datenbank ausgewählt ist aus der Gruppe, die aus Gensequenzdaten, Proteinsequenzdaten, anatomischen Daten, biochemischen Daten, biophysikalischen Daten oder einem mathematischen Modell des Vorstehenden besteht.

10. Interaktives computer-implementiertes System gemäß Anspruch 1, bei welchem die binäre Datenstruktur eine Kombination wenigstens zweier elementarer Datenstrukturen mit wenigstens einem Übergang zwischen diesen ist.

11. Interaktives computer-implementiertes System gemäß Anspruch 1, bei welchem die binäre Datenstruktur eine Kombination wenigstens zweier elementarer Datenstrukturen mit wenigstens einer damit assoziierten Ratenkonstante ist.

12. Computer-implementiertes Verfahren zum Konstruieren computer-ausführbarer Modelle des dynamischen Verhaltens von biologischen Prozessen basierend auf mehreren Typen von biologischer Information auf zellulärem und subzellulärem Niveau, umfassend:

> a) Zugreifen auf wenigstens eine interne und externe Datenbank, welche die mehreren Typen biologischer Information enthält;
> b) Erzeugen wenigstens einer Datenstruktur, die ausgewählt ist aus der Gruppe, die aus einer elementaren Datenstruktur, einer binären Datenstruktur und einer Weg-Datenstruktur oder einer Kombination von diesen besteht, wobei jede der Datenstrukturen der Gruppe wenigstens ein mit ihr assoziiertes Attribut aufweist;
> c) Erzeugen einer hierarchischen Beschreibung eines biologischen Prozesses durch interaktives Betrachten und Bearbeiten von Datenstrukturattributen;
> d) Verwenden einer Gleichungserzeugungs-Maschine, um ein System von Differentialgleichungen zu erzeugen, welches ein symbolisches Modell des biologischen Prozesses beschreibt; und
> e) Verwenden einer Rechenmaschine, um das System mathematischer Gleichungen in ein computer-ausführbares Modell des dynamischen Verhaltens des biologischen Prozesses zu transformieren.

13. Computer-implementiertes Verfahren gemäß Anspruch 12, außerdem umfassend den Schritt des interaktiven Betrachtens wenigstens einer der Datenstrukturen, Attribute oder hierarchischen Beschreibungen des biologischen Systems.

14. Computer-implementiertes Verfahren gemäß Anspruch 12, bei welchem die symbolischen Modelle in dem System zum anschließenden Modellieren gespeichert werden können.

15. Computer-implementiertes Verfahren gemäß Anspruch 12, bei welchem wenigstens zwei symbolischen Modelle durch die Gleichungserzeugungsmaschine erzeugt werden und von dem System verkoppelt werden, um ein multi-dimensionales Modell eines zellulären oder subzellulären Prozesses herzustellen.

16. Computer-ausführbares Modell des dynamischen Verhaltens eines biologischen Prozesses, wie mit dem System von Anspruch 1 konstruiert, umfassend ein Modell von dynamischem zellulären Verhalten, das mathematisch aus der wenigstens einen hierarchischen Beschreibung des biologischen Prozesses erzeugt wird, wobei die hierarchische Beschreibung aus mehreren Datenstrukturen erzeugt wird, wobei jede Datenstruktur aus wenigstens einer Datenbank erzeugt wird, die eine interne und externe, biologische Information enthaltende Datenbank umfaßt, und wobei jede Datenstruktur wenigstens ein mit ihr assoziiertes Attribut aufweist und aus der Gruppe ausgewählt ist, die aus einer elementaren Datenstruktur, einer binären Datenstruktur, einer Weg-Datenstruktur oder einer Kombination hiervon besteht.

17. Computer-ausführbares Modell gemäß Anspruch 16, bei welchem die hierarchische Beschreibung weiter in ein symbolisches Modell des biologischen Prozesses transformiert wird.

18. Computer-ausführbares Modell gemäß Anspruch 17, außerdem umfassend computer-ausführbaren, aus dem symbolischen Modell erzeugten Code.

19. Computerprogramm-Produkt zum Ausführen des Verfahrens von Anspruch 12, wenn es auf einem Computer läuft, umfassend:

> a) eine Datenspeicherstruktur, die wenigstens eine, biologische Information enthaltende Datenbank umfaßt, wobei die biologische Information in wenigstens eine Datenstruktur eingerichtet ist, die ein mit ihr assoziiertes Attribut aufweist, und wobei die Datenstruktur aus der Gruppe ausgewählt ist, die aus einer elementaren Datenstruktur, einer binären Datenstruktur, einer Weg-Datenstruktur oder einer Kombination hiervon besteht;
> b) Code-Mittel zum Arbeiten auf der Datenstruktur, um ein System von Differentialgleichungen zu erzeugen, die ein symbolisches Modell eines biologischen Prozesses beschreiben, und
> c) Code-Mittel zum Arbeiten auf dem System von Differentialgleichungen, um ein computer-ausführbares Modell des dynamischen Verhaltens des biologischen Prozesses zu erzeugen.

20. Verfahren zum Speichern und Suchen biologischer Information in dem System gemäß Anspruch 1, umfassend die folgenden Schritte:

> a) Verwenden des computer-implementierten Systems, um auf Information aus wenigstens einer Datenbank, die eine interne und externe, biologische Information enthaltende Datenbank umfaßt, zuzugreifen;

b) Schaffen wenigstens einer Datenstruktur mit einem damit assoziierten Attribut aus der Information in der Datenbank, wobei die Datenstruktur aus der Gruppe ausgewählt ist, die aus einer elementaren Datenstruktur, einer binären Datenstruktur, einer Weg-Datenstruktur oder einer Kombination hiervon besteht; und

c) Einrichten der Datenstruktur, um eine hierarchische Beschreibung biologischer Information zu erzeugen.

21. Verfahren gemäß Anspruch 20, außerdem umfassend den Schritt des Speicherns der Datenstruktur in dem System.

22. Verfahren gemäß Anspruch 22 außerdem umfassend die Verwendung einer graphischen Benutzerschnittstelle zum Interagieren mit der Datenstruktur.

**Revendications**

1. Système interactif implémenté sur ordinateur pour construire des modèles exécutables par ordinateur du comportement dynamique de processus biologiques basés sur plusieurs types d'informations biologiques au niveau cellulaire et subcellulaire, comprenant :

a) au moins une base de données (11) comprenant une base de données internes et externes contenant lesdits plusieurs types d'informations qui est utilisée pour générer au moins une structure de données (17), dans laquelle la structure de données est choisie dans le groupe consistant en une structure de données élémentaires, une structure de données binaires, une structure de données de chemin d'accès, ou une combinaison de celles-ci, chacune des structures de données dudit groupe étant associée à au moins un attribut ;

b) une interface utilisateur graphique (23) pour visualiser et organiser interactivement des attributs de structures de données pour générer une description hiérarchique d'un processus biologique ;

c) un moteur de génération d'équations (24) opérant sur la structure de données pour générer un système d'équations différentielles décrivant un modèle symbolique du processus biologique ; et

d) un moteur de calcul (22) opérant sur le système d'équations différentielles pour générer un modèle exécutable par ordinateur du comportement dynamique du processus biologique.

2. Système interactif implémenté sur ordinateur selon la revendication 1, dans lequel le modèle symbolique peut être stocké dans le système pour une modélisation ultérieure.

3. Système interactif implémenté sur ordinateur selon la revendication 1, dans lequel au moins deux modèles symboliques sont générés par le moteur de génération d'équations et couplés ensemble par le système pour produire un modèle multidimensionnel d'un processus cellulaire ou subcellulaire.

4. Système interactif implémenté sur ordinateur selon la revendication 1 ou 3, dans lequel le modèle symbolique comprend au moins deux équations différentielles.

5. Système interactif implémenté sur ordinateur selon la revendication 1, dans lequel la structure de données est une structure de données anatomiques.

6. Système interactif implémenté sur ordinateur selon la revendication 5, dans lequel la structure de données anatomiques est ensuite modifiée pour former au moins un modèle cellulaire structural décrivant la forme d'une cellule et le placement particulier d'organites à l'intérieur de celle-ci.

7. Système interactif implémenté sur ordinateur selon la revendication 1, dans lequel les structures de données binaires et de chemin d'accès sont organisées comme des diagrammes de transition d'états.

8. Système interactif implémenté sur ordinateur selon les revendications 1 et 4, dans lequel au moins une description hiérarchique de fonction subcellulaire est ensuite modifiée pour générer un modèle exécutable par ordinateur.

9. Système interactif implémenté sur ordinateur selon la revendication 1, dans lequel la base de données est choisie dans le groupe consistant en des données de séquences géniques, des données de séquences protéiques, des données anatomiques, des données biochimiques, des données biophysiques, ou un modèle mathématique des susdites.

**10.** Système interactif implémenté sur ordinateur selon la revendication 1, dans lequel la structure de données binaires est une composition d'au moins deux structures de données élémentaires ayant au moins une transition entre elles.

**11.** Système interactif implémenté sur ordinateur selon la revendication 1, dans lequel la structure de données binaires est une composition d'au moins deux structures de données élémentaires ayant au moins une constante de vitesse associée à celles-ci.

**12.** Procédé implémenté sur ordinateur pour construire des modèles exécutables par ordinateur du comportement dynamique de processus biologiques basés sur plusieurs types d'informations biologiques au niveau cellulaire et subcellulaire, comprenant :

a) l'accès à au moins une base de données internes et externes contenant lesdits plusieurs types d'informations biologiques ;
b) la génération d'au moins une structure de données choisie dans le groupe consistant en une structure de données élémentaires, une structure de données binaires et une structure de données de chemin d'accès ou une combinaison de celles-ci, dans laquelle chacune des structures de données dudit groupe a au moins un attribut associé à celle-ci ;
c) la génération d'une description hiérarchique d'un processus biologique, en visualisant et organisant interactivement des attributs de structures de données ;
d) l'utilisation d'un moteur de génération d'équations pour générer un système d'équations différentielles décrivant un modèle symbolique du processus biologique ; et
e) l'utilisation d'un moteur de calcul pour transformer le système d'équations mathématiques en un modèle exécutable par ordinateur du comportement dynamique du processus biologique.

**13.** Procédé implémenté sur ordinateur selon la revendication 12, comprenant en outre une étape de visualisation interactive d'au moins un des structures de données, attributs ou descriptions hiérarchiques d'un système biologique.

**14.** Procédé implémenté sur ordinateur selon la revendication 12, dans lequel les modèles symboliques peuvent être stockés dans le système pour une modélisation ultérieure.

**15.** Procédé implémenté sur ordinateur selon la revendication 12, dans lequel au moins deux modèles symboliques sont générés par le moteur de génération d'équations et couplés ensemble par le système pour produire un modèle multidimensionnel d'un processus cellulaire ou subcellulaire.

**16.** Modèle exécutable par ordinateur du comportement dynamique d'un processus biologique tel que construit avec le système selon la revendication 1, comprenant un modèle de comportement cellulaire dynamique généré mathématiquement à partir d'au moins une description hiérarchique du processus biologique, dans lequel la description hiérarchique est générée à partir d'une pluralité de structures de données dans lesquelles chaque structure de données est générée à partir d'au moins une base de données comprenant une base de données internes et externes contenant des informations biologiques, et dans lesquelles chaque structure de données a au moins un attribut associé à celle-ci et est choisie dans le groupe consistant en une structure de données élémentaires, une structure de données binaires, une structure de données de chemin d'accès, ou une combinaison de celles-ci.

**17.** Modèle exécutable par ordinateur selon la revendication 16, dans lequel la description hiérarchique est ensuite transformée en un modèle symbolique du processus biologique.

**18.** Modèle exécutable par ordinateur selon la revendication 17, comprenant en outre un code exécutable par ordinateur généré à partir du modèle symbolique.

**19.** Produit programme informatique pour réaliser le procédé selon la revendication 12 lorsqu'il est exécuté sur un ordinateur, comprenant :

a) une structure de stockage de données comprenant au moins une base de données contenant des informations biologiques dans laquelle les informations biologiques sont organisées en au moins une structure de données ayant un attribut associé à celle-ci, et dans laquelle la structure de données est choisie dans le groupe consistant en une structure de données élémentaires, une structure de données binaires, une structure de données de chemin d'accès, ou une combinaison de celles-ci ;

b) un moyen de codage pour opérer sur la structure de données pour générer un système d'équations différentielles décrivant un modèle symbolique d'un processus biologique ; et

c) un moyen de codage pour opérer sur le système d'équations différentielles pour générer un modèle exécutable par ordinateur du comportement dynamique du processus biologique.

20. Procédé de stockage et de recherche d'informations biologiques dans le système selon la revendication 1, comprenant les étapes suivantes :

a) utilisation du système implémenté sur ordinateur pour accéder à des informations à partir d'au moins une base de données comprenant une base de données internes et externes contenant des informations biologiques ;

b) création d'au moins une structure de données ayant un attribut associé à celle-ci à partir des informations dans la base de données, dans laquelle la structure de données est choisie dans le groupe consistant en une structure de données élémentaires, une structure de données binaires, une structure de données de chemin d'accès, ou une combinaison de celles-ci ; et

c) organisation de la structure de données pour générer une description hiérarchique d'informations biologiques.

21. Procédé selon la revendication 20 comprenant en outre une étape de stockage de la structure de données dans le système.

22. Procédé selon la revendication 20 comprenant en outre l'utilisation d'une interface utilisateur graphique pour interagir avec la structure de données.

# FIG. 1

EP 1 171 841 B1

# FIG. 2

# FIG. 3

# FIG. 4

EDS 1

EDS 3

EDS 4

EDS 2

# FIG. 5

$$C_1 \underset{K_{21}(V)}{\overset{K_{12}(V)}{\rightleftharpoons}} O_2$$

# FIG. 6

EDS 1 ⟶ EDS 2

EDS 3

# FIG. 7

Structural Database — 15

3-D Image Data

Attribute List — 44

Geometry Modeling Engine — 42

Structural, Finite-Element Cell Models — 43

Attribute List — 45

GUI — 23

# FIG. 8